(19) Europäisches Patentamt
European Patent Office
Office européen des brevets

(11) Publication number: **0 081 877**
**B1**

(12) **EUROPEAN PATENT SPECIFICATION**

(45) Date of publication of patent specification: **30.04.86**

(51) Int. Cl.⁴: **C 07 K 7/06,** C 07 K 7/20, A 61 K 37/02

(21) Application number: **82201544.2**

(22) Date of filing: **06.12.82**

# (54) LH-RH antagonists.

(30) Priority: **10.12.81 US 329526**
**15.04.82 US 368702**

(43) Date of publication of application:
**22.06.83 Bulletin 83/25**

(45) Publication of the grant of the patent:
**30.04.86 Bulletin 86/18**

(84) Designated Contracting States:
**AT BE CH DE FR GB IT LI LU NL SE**

(56) References cited:
**EP-A-0 041 286**
**US-A-4 317 815**

**Chemical Abstracts vol. 94, no. 5, 2 February 1981, Columbus, Ohio, USA, D.H. COY et al. "LH-RH antagonists with potent antivulatory activity", abstract no. 25416u**
**J. Med. Chem. 18/12 (1975), S. 1247**
**J. Med. Chem. 21/3 (1978), S. 276**

**The file contains technical information submitted after the application was filed and not included in this specification**

(73) Proprietor: **COY, David Howard**
**4319 Perrier Street**
**New Orleans Louisiana 70115 (US)**
(73) Proprietor: **Schally, Andrew Victor**
**5025 Kawanee Avenue**
**Metairie Louisiana 70002 (US)**

(72) Inventor: **COY, David Howard**
**4319 Perrier Street**
**New Orleans Louisiana 70115 (US)**
Inventor: **Schally, Andrew Victor**
**5025 Kawanee Avenue**
**Metairie Louisiana 70002 (US)**

(74) Representative: **Hermans, Franciscus G.M. et al**
**Postbus 20**
**NL-5340 BH Oss (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 081 877 B1

**Description**

This invention relates to novel peptides which are antagonist of the luteinizing hormone releasing hormone (LH-RH), which has the structure:

$$p\text{-}Glu\text{-}His\text{-}Trp\text{-}Ser\text{-}Tyr\text{-}Gly\text{-}Leu\text{-}Arg\text{-}Pro\text{-}Gly\text{-}NH_2.$$

More specifically, this invention relates to luteinizing hormone releasing factor (LH-RH) analogs, salts thereof, to processes and intermediates for preparing these analogs, and to pharmaceutical compositions pertaining to these analogs.

The LH-RH analogs of this invention differ in structure from LH-RH by having the amino acid residues at positions 1, 2 and 6, and optionally at positions 3 and 10, replaced by other amino acid residues.

For several years investigators have been searching for selective, potent antagonists of the LH-RH decapeptide. See the review article by D. H. Coy and A. V. Schally, Annals of Clinical Research, *10*, 139 (1978). The high degree of interest in such antagonists is due to their usefulness in the endocrine and cancer fields.

A great number of compounds have been prepared as potential LH-RH antagonist but most of these compounds lack potency or are mixed agonist and antagonist of the LH-RH decapeptide. The most interesting antagonists to date have been compounds having a modified structure of LH-RH. For instance, [D-$Phe^2$]-LH-RH, R. A. W. Rees *et al.*, J. Med. Chem., *17*, 1016 (1974); [D-$Phe^2$, D-$Phe^6$]-LH-RH, [D-$Phe^2$, $Phe^3$, D-$Phe^6$]-LH-RH and [D-$Phe^2$, D-$Trp^3$, D-$Phe^6$]-LH-RH, D. H. Coy *et al.*, in "Peptides 1976", A. Loffet, Ed., Editions de l'Université de Bruxelles, Brussels, Belgium, 1977, p. 463; [D-p-F-$Phe^2$-D-$Ala^6$]-LH-RH, C. W. Beattie *et al.*, J. Med. Chem., *18*, 1247 (1975) and [Ac-D-$Phe^1$, D-$Phe^2$, D-$Trp^{3,6}$]-LH-RH, K. Channabasavaiah and J. M. Stewart, Biochem. Biophys. Res. Commun., *86*, 1266 (1979) and the peptides disclosed in the U.S. patent specification No. 4,317,815.

The latter prior art reference (U.S. patent No. 4,317,815) describes the most active LH-RH antagonists known at the filing date of the present application. Amongst these most active prior art LH/RH antagonists the following peptides show a 100% inhibition of ovulation at a dose level of about *15* μg:

N-Ac-D-$Phe^1$, D-pCl-$Phe^2$, D-$Trp^3$, D-$Trp^6$-D-$Ala^{10}$LH/RH

and

N-Ac-D-pCl-$Phe^{1,2}$, D-$Trp^3$, D-$Phe^6$, D-$Ala^{10}$ LH/RH.

It was, indeed, found unexpectedly that replacement of D-$Trp^6$ or D-$Phe^6$ in these most active prior art peptides, with a basic amino acid, such as D-Lys, and particularly D-Arg, further increase the activity up to a dose level of about *1 to 3* μg, as is clearly illustrated in the tables I, II and III of the present specification.

Such dramatic increase of activity could not be expected or foreseen, the more so as D-$Lys^6$ and D-$Arg^6$ were already suggested as possible substitution candidates in other LHRH analog series of peptides without favourable results. The latter may be illustrated by the following data.

Beattie et al. J. Med. Chem. *18*(12) 1247, 1975 (see table II at page 1248):

| Peptide | Dose mg | % Anti-ovulatory effect |
|---|---|---|
| ref.: D-$Phe^2$, *D-$Phe^6$* LHRH | 6 | 80% inhibition |
| D-$Phe^2$-D-$Lys^6$ LHRH | 6 | 0% inhibition |
| D-$Phe^2$-D-$Arg^6$ LHRH | 4,5 | 40% inhibition |

Seprodi et al. J. Med. Chem. *21*(3), 276 (1978) (see table IV, at page 278):

| Peptide (LH/RH analog) | Dose mg | % Anti-ovulatory effect |
|---|---|---|
| ref.: D-$Phe^2$, D-$Trp^3$, D-$Phe^6$ LH/RH | 1 | 83% inhibition |
| D-$Phe^2$, D-$Trp^3$, D-$Lys^6$ LH/RH | 1 | 0% inhibition |

The replacement of D-Phe[6] by D-Lys[6] or D-Arg[6] in the above LH-RH analogs is clearly unfavourable and does not encourage such like replacement in other LH-RH series.

The present invention provides pure LH-RH antagonists that are more potent than any of the LH-RH antagonists reported to date; and moreover exert their activity also when administered through the oral route.

The compounds of this invention, i.e. the LH-RH analogs, are represented by formula I

$$X\text{-}R^1\text{-}R^2\text{-}R^3\text{-Ser-Tyr-}R^4\text{-Leu-Arg-Pro-}R^5\text{-}NH_2 \qquad (I)$$

in which X is hydrogen, lower alkanoyl, HOOC—$(CH_2)_n$—CO wherein n is an integer from 2 to 6, $R^1$ is Gly, L-Ala, L-3-(1-naphtyl)-Ala, L-3-(2-naphtyl)-Ala, D-Ala, D-3-(1-naphtyl)-Ala, D-3-(2-naphtyl)-Ala, D-Trp, D-Phe or D-Phe having one or more substituents at the phenyl moiety selected from halogen, nitro, amino, alkyl (1—4 C), cyano, trifluoromethyl, hydroxy and alkoxy (1—4 C); $R_2$ is D-Phe having one or two substituents at the phenyl moiety, one substituent being always in para position, which substituent(s) is (are) selected from the group consisting of halogen, nitro, amino, alkyl (1—4 C), cyano, trifluoromethyl, hydroxy and alkoxy (1—4 C); $R^3$ is D-Trp, L-Trp, L-Phe or L- or D-3-(2-naphtyl)-Ala; $R^4$ is D-Lys, D-Arg, D-Orn, D-homo-Arg or D-His and $R^5$ is Gly or D-Ala.

A preferred group of compounds of formula I is one in which X is hydrogen, lower alkanoyl, HOOC—$(CH_2)_n$—CO wherein n is an integer from 2 to 6, $R^1$ is D-3-(2-naphtyl)-Ala, L-3-(2-naphtyl)-Ala, D-Trp, D-Phe or D-p-Y-Phe wherein Y is selected from the group consisting of halo, nitro, amino, methyl, cyano, trifluoromethyl, hydroxy and methoxy; $R^2$ is D-p-Y-Phe in which Y is as defined herein; $R^3$ is D-Trp; $R^4$ is D-Lys or D-Arg and $R^5$ is D-Ala.

Another preferred group of the compounds of formula I is that in which X is hydrogen, lower alkanoyl or HOOC—$(CH_2)_n$—CO wherein n is as defined herein: $R^1$ is D-3-(2-naphtyl)-Ala, D-Trp, D-Phe or D-p-halo-Phe; $R^2$ is D-p-halo-Phe; $R^3$ is D-Trp; $R^4$ is D-Lys or D-Arg and $R^5$ is D-Ala.

Still another preferred group of the compounds of formula I is that in which X is acetyl or $HOOCCH_2CH_2CO$; $R^1$ is D-3-(2-naphtyl)-Ala, D-Trp, D-Phe or D-p-Cl-Phe; $R^2$ is D-p-Cl-Phe; D-p-Br-Phe or D-p-F-Phe; $R^3$ is D-Trp; $R^4$ is D-Lys or D-Arg and $R^5$ is D-Ala.

The therapeutically acceptable salts of the compound of formula I are included within the scope of this invention.

The compounds of formula I are prepared by conventional peptide synthesis or by solid phase techniques.

In general, the peptides I may be prepared by removal of the protective group(s) from a peptide of formula IA:

$$X^1\text{-}R^1\text{-}R^2\text{-}R^3\text{-Ser}(R^6)\text{-Tyr}(R^7)\text{-}R^{4(1)}\text{-Leu-Arg}(N^G\text{-}R^8)\text{-Pro-}R^5\text{-}NH_2$$

wherein

$R^1$, $R^2$, $R^3$ and $R^5$ are as herein defined,

$X^1$ has the same meaning as X as herein defined but in addition may represent an N-protective group or an N-protected acyl portion of a D- or L-amino acid, $R^{4(1)}$ has the same meanings as $R^4$ defined already, but may in addition also represent $N^\varepsilon$-protected D-lysyl, NΔ-protected D-ornithyl, $N^G$-protected D-arginyl, $N^G$-protected D-homo arginyl or N-protected histidyl, and $R^6$, $R^7$ and $R^8$ are each hydrogen or a protective group, with the proviso that at least one of the radicals $X^1$, $R^6$, $R^7$, $R^8$ or $R^{4(1)}$ is or contains a protective group, and, if desired, followed by N-acylation of the resulting compound of formula I, wherein X is hydrogen.

The compounds of formula I may also be prepared by a process, which comprises:

a) reacting a compound of the formula II:

$$X\text{-}R^1\text{-}R^2\text{-}R^3\text{-Ser}(R^6)\text{-Tyr}(R^7)\text{-}R^{4(1)}\text{-Leu-Arg}(N^G\text{-}R^8)\text{-Pro-}R^5\text{-A} \qquad (II)$$

wherein

X, $R^1$, $R^2$, $R^3$, $R^{4(1)}$, $R^5$, $R^6$, $R^7$ and $R^8$ are as defined herein and A is

NHCHPh—pH—[Resin support]

with a reagent capable of cleaving off the resin support and the anchoring radical, i.e.

—CHPh—Ph—[resin support]

or

b) subjecting the compound of the formula II wherein X, $R^1$, $R^2$, $R^3$, $R^{4(1)}$, $R^5$, $R^6$, $R^7$ and $R^8$ are as defined herein and A is

O—$CH_2$—Ph—[resin support]

to ammonolysis, to obtain in both cases (a and b) a peptide of the formula III:

$$X\text{-}R^1\text{-}R^2\text{-}R^3\text{-Ser}(R^6)\text{-Tyr}(R^7)\text{-}R^{4(1)}\text{-Leu-Arg}(N^G\text{-}R^8)\text{-Pro-}R^5\text{-}NH_2 \qquad \text{(III)}$$

in which

X, $R^1$, $R^2$, $R^3$, $R^{4(1)}$, $R^5$, $R^6$, $R^7$ and

$R^8$ are as defined herein; followed by, when the compound of formula III is different from the compound of formula I, reacting the compound of formula III with a reagent capable of removing the protecting groups without affecting the compound of formula I.

The compounds of formulae IA and II, and the compound of formula III when different from the compound of formula I, also are included within the scope of this invention as intermediates in the synthesis of the peptides of formula I.

A further aspect of the invention relates to intermediates linked to a solid resin support and having a protected α-amino group. These intermediates are represented by the formula IV:

$$R^9\text{-}R^1\text{-}R^2\text{-}R^3\text{-Ser}(R^6)\text{-Tyr}(R^7)\text{-}R^{4(1)}\text{-Leu-Arg}(N^G\text{-}R^8)\text{-Pro-}R^5\text{-A}$$

in which $R^1$ to $R^8$, inclusive, are as defined herein and $R^9$ is an α-amino protective group known to be useful in the art of the stepwise synthesis of polypeptides, suitable groups being listed hereinafter, and A is selected from the group consisting of

NH—CHPh—Ph—[resin support]

and

$OCH_2$—Ph—[resin support]

A gonadotropin antagonizing pharmaceutical composition is provided by admixing the compound of formula I with a pharmaceutically acceptable carrier.

The term "alkanoyl" or "lower alkanoyl" as used herein means straight chain alkanoyl radicals containing one to six carbon atoms, e.g. formyl, acetyl, propionyl, butyryl and hexanoyl, and branched chained alkanoyl radicals containing four to six carbon atoms, e.g. isobutyryl and pivaloyl.

The term "lower alkanoic acid" as used herein means both straight and branched chain alkanoyl radicals containing two to six carbon atoms and includes acetic acid, propionic acid, pivalic acid, hexanoic acid and the like.

The term "organic proton acceptor" as used herein means the organic bases, or amines for instance, triethylamine, pyridine, N-ethylmorpholine, imidazole and the like.

The term "halo" or "halogen" includes chlorine, bromine, fluorine and iodine.

The term "amino acid" as used herein means the well known and reasonably accessible amino acids which are described in general textbooks on peptide chemistry; for instance, see K. D. Kopple, "Peptides and Amino Acids", W. A. Benjamin Inc., New York and Amsterdam, 1966, pp. 4—7.

The term "acyl portion" of an amino acid means a radical derived from the corresponding amino acid by eliminating the hydroxyl of the carboxy group.

The term "amino acid residue" refers to a radical derived from the corresponding amino acid by eliminating the hydroxyl of the carboxy group and one hydrogen of the amino group.

$N^G$ means the side chain nitrogen atoms of arginine.

The symbol "Ph-" means phenyl and the symbol -Ph- means 1,4-phenylene.

"Ac" means acetyl. "Succ" means HOOC—$(CH_2)_2$—CO, i.e. succinyl, a 3-carboxy-1-propionyl radical.

"A" is an anchoring bond linked to a solid resin (resin support) used in solid phase synthesis and is selected from the class consisting of:

—NH—CHPh—Ph—[resin support]

and

$OCH_2$—Ph—[resin support].

The term "anchoring radical" means that portion of A which is —CH—Ph—Ph— or —$CH_2$—Ph.

In general, the abbreviations used herein for designating the amino acids and the protective groups are based on recommendations of the IUPAC—IUB Commission on Biochemical Nomenclature, see Biochemistry II, 1726 (1972). For instance, t-Boc represents t-butyloxycarbonyl, Z represents benzyloxycarbonyl, Tos represents tosyl and Bzl represents benzyl. The abbreviations used herein for the various amino acids are Ala, alanine; Lys, lysyl, Arg, arginine, Gly, glycine; Leu, leucine; Orn, ornithyl, Phe, phenylalanine; Pro, proline; Ser, serine; Trp, tryptophan; and Tyr, tyrosine. All amino acids described herein are in the L-series unless stated otherwise, e.g. D-Ala is a D-alanyl residue, D-Phe is a D-phenylalanyl residue and D-Trp is a D-tryptophyl residue.

The LH-RH analogs of this invention can be obtained in the form of an acid addition salt. Examples of salts are those with organic acids, e.g. acetic, lactic, succinic, benzoic, salicylic, methanesulphonic or p-toluenesulphonic acid, as well as polymeric acids, such as tannic acid or carboxymethyl cellulose, and

salts with inorganic acids, such as hydrohalic acids, e.g. hydrochloric acid, or sulphuric acid, or phosphoric acid. If desired, a particular acid addition salt is converted into another acid addition salt, e.g. a salt with a non-toxic, therapeutically acceptable acid, by treatment with the appropriate ion exchange resin in the manner described by R. A. Boissonnas, *et al.*, Helv. Chim. Acta., *43*, 1349 (1960). Suitable ion exchange resins are cellulose based cation exchangers, for example carboxymethylcellulose or chemically modified, cross linked dextran cation exchangers, for example the chemically modified, cross-linked dextran cation exchanger sold under the trade mark Sephadex C, and strongly basic anion exchange resins, for example those listed by J. P. Greenstein and M. Winitz in "Chemistry of the Amino Acids", John Wiley and Sons, Inc. New York and London, 1961, Vol. 2, p. 1456. These therapeutically acceptable acid addition salts are included within the scope of this invention.

The compounds of formula I in which X is HOOC—$(CH_2)_n$—CO wherein n is an integer from 2 to 6 form salts with suitable therapeutically acceptable inorganic and organic bases. These derived salts possess the same activity as the parent acid and are included within the scope of this invention. The acid is transformed in excellent yield into the corresponding therapeutically acceptable salt by neutralization of said acid with the appropriate inorganic or organic base. Suitable inorganic bases to form these salts include, for example, the hydroxides, carbonates, bicarbonates or alkoxides of the therapeutically acceptable alkali metals or alkaline earth metals, for example, sodium, potassium, magnesium, calcium and the like. Suitable organic bases include the following amines; lower mono-, di- and trialkylamines, the alkyl radicals of which contain up to three carbon atoms, such as methylamine, dimethylamine, trimethylamine, ethylamine, di- and triethylamine, N-methyl-N-ethylamine, and the like; and mono-, di- and trialkanolamines, the alkanol radicals of which contain up to three carbon atoms, for example, mono-, di- and triethanolamine.

With reference to the compound of formula II,

$$X\text{-}R^1\text{-}R^2\text{-}R^3\text{-Ser}(R^6)\text{-Tyr}(R^7)\text{-}R^{4(1)}\text{-Leu-Arg}(N^G\text{-}R^8)\text{-Pro-}R^5\text{-A},$$

and the compound of formula III,

$$X\text{-}R^1\text{-}R^2\text{-}R^3\text{-Ser}(R^6)\text{-Tyr}(R^7)\text{-}R^{4(1)}\text{-Leu-Arg}(N^G\text{-}R^8)\text{-Pro-}R^5\text{-}NH_2,$$

in a preferred embodiment X, $R^1$, $R^2$, $R^3$, $R^5$ and A are as defined herein, $R^{4(1)}$ represents D-Lys($N^\varepsilon$-$R^{10}$), D-Orn($N^\delta$-$R^{10}$), D-Arg($N^G$-$R^8$), D-homo Arg($N^G$-$R^8$) or D-His($N^I$-$R^{11}$), $R^6$ is a protective group for the hydroxyl group of serine and is selected from the group of 2-bromo-benzyloxy-carbonyl, benzyl, acetyl, tosyl, benzoyl, tert.butyl, tetrahydropyra-2-yl, trityl, 2,4-dichlorobenzyl and benzyloxycarbonyl; $R^7$ is hydrogen or a protective group for the hydroxyl of tyrosine selected from the group defined hereinbefore for $R^6$, $R^8$ is a protective group for the $N^\delta$, $N^\omega$, and $N^\omega$ nitrogen atoms of arginine selected from the group consisting of tosyl, nitro, benzyloxycarbonyl and adamantyloxycarbonyl, $R^{10}$ is a protective group for the ε-amino group of lysine or the δ-amino group of ornithine and is preferably selected from tosyl, tert.butyloxycarbonyl and 2-halo-benzyloxycarbonyl, and $R^{11}$ is H or a protective group for the imidazol group of histidine and is preferably selected from benzyl, dinitrophenyl and trityl. In another preferred embodiment with reference to the compounds IA, II and III, the D-histidyl (if present), seryl and tyrosyl residues are not protected, the arginine residue(s) is(are) protected in the form of a strong acid addition salt, e.g., the hydrochloric acid, p-toluenesulphonic acid or sulphuric acid addition salt, and the D-lysine or D-ornithine residues (if present) are protected by a 2-chloro or 2-bromo benzyloxycarbonyl group.

The valuable LH-RH antagonizing property of the compounds of this invention are demonstrated by standard pharmacological procedures. For example, this activity can be demonstrated in the test described by A. de la Cruz *et al.*, Science, *191*, 195 (1976). More explicitly, the assay is performed using mature female rats (Charles River Breeding Laboratories, Boston, Mass., U.S.A.) weighing about 200 g and exhibiting normal four-day cycles. The analogs are administered subcutaneously in 20% or 40% propylene glycol in physiologic saline or as a suspension in corn oil at 12 noon of the day of proestrus. On the following day, the rats are sacrificed, their fallopian tubes and uteri flushed with saline and the washes examined for ova.

The Tables I and II show the activity of some peptides of the invention, administered subcutaneously, in comparison with the reference peptide known from the U.S. patent specification No. 4,317,815.

Table I shows the anti-ovulatory activity using 40% propylene glycol in saline as the carrier substance. (The ref. peptide is in 20% propylene glycol in saline).

TABLE I

| Peptides | Dose in µg s.c. | % Blockade of ovulation |
|---|---|---|
| N-Ac-[D-p-Cl-$Phe^{1,2}$, D-$Trp^3$, D-$Phe^6$, D-$Ala^{10}$]-LH-RH | 7.5 | 88 |
| (ref. USP 4,317,815) | 3 | 0 |
| N-Ac-[D-p-Cl-$Phe^{1,2}$, D-$Trp^3$ D-$Lys^6$, D-$Ala^{10}$]-LH-RH | 15<br>7.5<br>3 | 100<br>100<br>50 |
| N-Ac-[D-p-Cl-$Phe^{1,2}$, D-$Trp^3$ D-$Arg^6$, D-$Ala^{10}$]-LH-RH | 5<br>3 | 100<br>67 |
| N-Ac-[D-p-Cl-$Phe^{1,2}$, D-$Trp^3$ D-$His^6$, D-$Ala^{10}$]-LH-RH | 3 | 22 |
| N-Ac-[D-3-(2-naphtyl)-$Ala^1$, D-p-Cl-$Phe^2$, D-$Trp^3$, D-$Arg^6$, D-$Ala^{10}$]-LH-RH | 3<br>1 | 100<br>77 |

Table II shows the anti-ovulatory activity using a suspension of the peptide in *Corn oil.*

TABLE II

| Peptides | Dose in µg s.c. | % Blockade of ovulation |
|---|---|---|
| N-Ac-[D-$Phe^{1,2}$, D-$Trp^{3,6}$]-LH-RH (ref. Biochem. Biophys. Res. Commun. *86*, 1226, 1979) | 250<br>100 | 100<br>40 |
| N-Ac-[D-p-Cl-$Phe^{1,2}$, D-$Trp^3$ D-$Phe^6$, D-$Ala^{10}$]-LH-RH | 3 | 0 |
| (ref. USP 4,317,815) | | |
| N-Ac-[D-p-Cl-$Phe^{1,2}$, D-$Trp^3$, D-$Arg^6$, D-$Ala^{10}$]-LH-RH | 3<br>1.5<br>0.750 | 100<br>90<br>40 |
| N-Ac-[D-p-Cl-$Phe^{1,2}$, D-$Trp^3$, D-$Lys^6$, D-$Ala^{10}$]-LH-RH | 3 | 70 |

In contrast to prior art peptides the present peptides possess also surprisingly potent oral activity. In order to investigate the oral anti-ovulatory activity, rats were starved for 24 hours prior to the administration of the peptide to be tested. The peptide is dissolved or emulgated in a suitable vehicle; one ml solution or emulsion was given by stomach tube.

TABLE III

Anti-ovulatory activity of the reference peptide N-Ac-[D-p-Cl-Phe[1,2], D-Trp[3], D-Phe[6]-D-Ala[10]]-LH-RH (US—A—4317815) administered by gavage: 2 mg/ml/rat at 9.30 AM of pro-estrus

| Vehicle | % Inhibition of ovulation |
|---|---|
| 40% Propylene glycol in lipomul oral and 1000 Int. Units trasylol | 0 |
| 40% Propylene glycol in coconut oil and 1000 Int. Units trasylol | 0 |

Anti-ovulatory activity of the peptide of this invention: N-Ac-[D-p-Cl-Phe[1,2], D-Trp[3], D-Arg[6], D-Ala[10]]-LH-RH administered by gavage: 2 mg/ml/rat at 2 PM of pro-estrus

| Vehicle | % Inhibition of ovulation |
|---|---|
| 40% Propylene glycol in saline and 1000 Int. Units of trasylol | 100 |
| 40% Propylene glycol in coconut oil and 1000 Int. Units of trasylol | 100 |

The LH-RH antagonizing properties of the peptides of this invention make the compounds useful in human and veterinary practice. For instance, the compounds of formula I find use as agents for relieving the complications from the undesirable physiological availability of pituitary gonadotropins in a mammal. Such complications include precocious puberty; hormone dependent tumors such as malignant and benign prostatic tumors, and mammary, ovarian and testicular tumors; hirsutism; acne; amenorrhea, e.g. secondary amenorrhea; endometriosis, and ovarian and mammary cystic diseases in both animals and humans. The compounds of formula I also are useful for regulating ovulation, thus rendering them useful agents for controlling fertility, e.g. as precoital or postcoital contraceptives, for synchronizing estrus in livestock and for improving the "rhythm" method. Also, the compounds are useful for regulating the human menopausal gonadotropin, follicle-stimulating hormone (FSH) and luteinizing hormone (LH) during perimenopausal and postmenopausal periods in women.

When the compound of formula I, preferably in the form of an acid addition salt, is employed in human or veterinary medicine, it is administered systemically, either orally or by subcutaneous or intramuscular injection, or by sublingual, nasal, or vaginal administration, in compositions in conjunction with a pharmaceutically acceptable vehicle or carrier.

The daily dosage of the compounds of formula I will vary with the form of administration and with the particular patient under treatment. Generally, treatment is initiated with small dosages substantially less than the optimum dose of the compound. Thereafter, the dosage is increased by small increments until the optimum effect under the circumstances is reached. In general, the compound obtained by the process is most desirably administered at a concentration level that generally will inhibit release of LH and of FSH without causing any harmful or deleterious side effects, and preferably at a level that is in a range of from 1 µg to about 1000 µg per kilogram bodyweight for parenteral administration and from 0.5 mg to about 100 mg per kilogram bodyweight for enteral administration. However, a dosage level for parenteral administration that is in the range of from 2.5 µg to about 250 µg and for enteral administration of from 1 mg to 25 mg is most desirably employed in order to achieve effective results.

For administration by the nasal route as drops or spray it is preferred to use the compound of formula I in solution in a sterile aqueous vehicle which may also contain other solutes such as buffers or preservatives, as well as sufficient quantities of pharmaceutically acceptable salts or of glucose to make the solution isotonic. Doses by the intranasal route range from 100 µg to 10.0 mg/kg, or preferably 100 µg to 1.0 mg/kg of body weight.

The compound of formula I also may be administered as nasal or vaginal powders or insufflations. For such purposes the decapeptide is administered in finely divided solid form together with a pharmaceutically acceptable solid carrier; for example, a finely divided polyethylene glycol for instance the polyethylene glycol sold under the trade mark "Carbowax 1540"; finely divided lactose; or preferably for vaginal administration, very finely divided silica, for instance, the silica sold under the trade mark "Cab-O-Sil". Such compositions may also contain other excipients in finely divided solid form such as preservatives, buffers, or surface active agents.

For sublingual or vaginal administration, the compound is formulated preferably in solid dosage forms such as sublingual tablets or vaginal inserts or suppositories with sufficient quantities of solid excipients such as starch, lactose, certain types of clay, buffers, and lubricating, disintegrating, or surface-active agents, or with semi-solid excipients commonly used in the formulation of suppositories. Examples of such excipients are found in standard pharmaceutical texts, e.g. in Remington's Pharmaceutical Sciences, Mack Publishing Company, Easton, Pa., 1970.

It is often desirable to administer the compounds of formula I continuously over prolonged periods of time in long-acting, slow-release or depot dosage forms. Such dosage forms may either contain a pharmaceutically acceptable salt of the compound having a low degree of solubility in body fluids, for example one of those salts described below, or they may contain the compound in the form of a water-soluble salt together with a protective carrier which prevents rapid release. In the latter case, for example, the compound may be formulated with a non-antigenic partially hydrolyzed gelatin in the form of a viscous liquid; or it may be adsorbed on a pharmaceutically acceptable solid carrier, for example, zinc hydroxide, and may be administered in suspension in a pharmaceutically acceptable liquid vehicle; or the decapeptide may be formulated in gels or suspensions with a protective non-antigenic hydrocolloid, for example sodium carboxymethylcellulose, polyvinylpyrrolidone, sodium alginate, gelatine, polygalacturonic acids, for example, pectin, or certain mucopolysaccharides, together with aqueous or non-aqueous pharmaceutically acceptable liquid vehicles, preservatives, or surfactants. Examples of such formulations are found in standard pharmaceutical texts, e.g. in Remington's Pharmaceutical Sciences, cited above. Long-acting, slow-release preparations of the compound may also be obtained by micro-encapsulation in a pharmaceutically acceptable coating material, for example gelatine, polyvinyl alcohol or ethyl cellulose. Further examples of coating materials and of the processes used for microencapsulation are described by J. A. Herbig in "Encyclopedia of Chemical Technology", Vol. 13, 2nd ed., Wiley, New York, 1967, pp. 436—456. Such formulations, as well as suspensions of salts of the compound which are only sparingly soluble in body fluids, are designed to release from about 1—1000 mcg of the compound per kilogram body weight per day, and are preferably administered by intramuscular injection. Alternatively, some of the solid dosage forms listed above, for example certain sparingly water-soluble salts or dispersions in or adsorbates on solid carriers of salts of the compound, for example dispersions in a neutral hydrogel of a polymer of ethylene glycol methacrylate or similar monomers, cross-linked as described in U.S. Patent 3,551,556, issued December 29, 1970 to K. Kliment, *et al.*, may also be formulated in the form of pellets releasing about the same amounts as shown above and may be implanted subcutaneously or intramuscularly.

Alternatively, slow-release effects over prolonged periods of time may also be obtained by administering the compound obtained by the process of this invention in an intra-vaginal device or in a temporary implant, for example a container made of a non-irritating silicone polymer such as polysiloxane, a suitable polysiloxane is sold under the trade mark "Silastic", or of a neutral hydrogel of a polymer as described above, possessing the required degree of permeability to release from about 0.1 µg to about 50 µg per kilogram body weight per day. Such intravaginal or implant dosage forms for prolonged administration have the advantage that they may be removed when it is desired to interrupt or to terminate treatment.

Chemical synthesis

In selecting a particular side chain protective group to be used in the synthesis of the present decapeptides, the following rules should be followed:

(a) the protective group must be stable to the reagent and under the reaction conditions selected for removing the α-amino protective group at each step of the synthesis,

(b) the protective group must retain its protecting properties (i.e., not be split off under coupling conditions), and

(c) the side chain protective group must be removable upon the completion of the synthesis of the peptide containing the desired amino acid sequence under reaction conditions that will not alter the peptide chain.

With reference to α-amino protective groups, for instance $R^9$, suitable protective groups include (1) aliphatic urethan protective groups illustrated by t-butyloxycarbonyl, diisopropylmethoxycarbonyl, biphenylisopropyloxycarbonyl, isopropyloxycarbonyl, t-amyloxycarbonyl, ethoxycarbonyl and allyloxycarbonyl; (2) cycloalkyl urethan type protective groups illustrated by cyclopentyloxycarbonyl, adamantyloxycarbonyl, d-isobornyloxycarbonyl and cyclohexyloxycarbonyl; and nitrophenylsulfenyl; α,α - dimethyl - 3,5 - dimethoxybenzyloxycarbonyl; and trityl. The preferred α-amino protective groups are selected from the group consisting of t-butyloxycarbonyl, cyclopentyloxycarbonyl, t-amyloxycarbonyl, d-isobornyloxycarbonyl, o-nitrophenylsulfenyl, biphenylisopropyloxycarbonyl, and α,α - dimethyl - 3,5 - dimethoxybenzyloxycarbonyl.

Using solid phase techniques the synthesis is commenced from the C-terminal end of the peptide using an α-amino protected amino acid linked to a solid resin. Such a starting material is prepared for example, by attaching an α-amino protected glycine to a benzhydrylamine resin, a chloromethylated resin or a hydroxymethyl resin, the former being preferred. The preparation of a benzhydrylamine resin is described by P. Rivaille, *et al.*, Helv. Chim. Acta., *54*, 2772 (1971) and the preparation of the hydroxymethyl

resin is described by M. Bodanszky and J. T. Sheehan, Chem. Ind. (London), *38*, 1597 (1966). A chloromethylated resin is commercially available from Bio Rad Laboratories, Richmond, California. In using the benzhydrylamine resin, an amide anchoring bond is formed with the α-amino protected glycine or D-alanine, illustrated as follows for glycine:

$$R^9{-}NH{-}CH_2{-}\overset{\overset{\displaystyle O}{\|}}{C}{-}NH{-}CHPh{-}Ph{-}\boxed{\text{resin support}}$$

This permits the C-terminal amide function to be obtained directly after the amino acid sequence in the synthesis is completed by cleaving off the resin support and anchoring radical of the linked peptide to form the amino acid amide at the C-terminal portion of the desired compound. In this instance the use of hydrogen fluoride for cleaving off the resin support also removes the side chain protective groups to give the decapeptide of this invention.

When the other resins are used, the anchoring bond is the benzylester group as illustrated hereinbefore. In this instance a convenient procedure for converting the linked protected peptide to the C-terminal amide is to ammonolize the protected peptide off the resin and then remove the protective groups of the resulting amide by treatment with sodium in liquid ammonia or by the hydrogen fluoride cleavage. An alternative procedure would be to cleave by transesterification with a lower alkanol, preferably methanol or ethanol, in the presence of triethylamine and then convert the resulting ester into an amide and subsequently deprotect as described above. See also J. M. Steward and J. D. Young, "Solid Phase Peptide Synthesis", W. H. Freeman & Co., San Francisco, 1969, pp. 40—49.

More specifically, in an embodiment of the present invention, an α-amino protected amino acid; namely, an α-amino protected glycine, preferably t-butyloxycarbonylglycine; or an α-amino protected D-alanine, preferably t-butyloxycarbonyl-D-alanine; is coupled to the benzhydrylamine resin with the aid of a carboxy group activating compound, preferably, dicyclohexylcarbodiimide or diisopropylcarbodiimide. Following the coupling of the α-amino protected amino acid to the resin support, the α-amino protecting group is removed such as by using trifluoroacetic acid in methylene chloride, trifluoroacetic acid alone or hydrochloric acid in dioxane. The deprotection is carried out at a temperature between 0°C and room temperature (i.e. 20 to 24°C). Other standard cleaving reagents and conditions for removal of specific α-amino protecting groups may be used as described by E. Schroder and K. Lubke, "The Peptides", Vol. I, Academic Press, New York, 1965, pp. 72—75. After removal of the α-amino protecting group, the remaining α-amino protected amino acids are coupled step-wise in the desired order to obtain the peptide. Each protected amino acid is introduced into the solid phase reactor in about a three-fold excess and the coupling is carried out in a medium of methylene chloride or mixtures of dimethyl-formamide in methylene chloride. In cases where incomplete coupling occurred, the coupling procedure is repeated before removal of the α-amino protecting group, prior to the coupling of the next amino acid to the solid phase reactor. The success of the coupling reaction at each stage of the synthesis is monitored by the ninhydrin reaction as described by E. Kaiser, *et al.*, Analyt. Biochem., *34*, 595 (1970). In this manner the compounds of formula II in which X is hydrogen is obtained.

After the desired amino acid sequence has been synthesized, the peptide is removed from the resin support by treatment with a reagent such as hydrogen fluoride which not only cleaves the peptide from the resin but also cleaves all remaining side chain protecting groups to obtain directly the peptide of formula I wherein X is hydrogen in the case where the benzhydrylamine resin was used.

Where a benzylester resin is used the peptide may be separated from the resin by transesterification with a lower alkanol, preferably methanol or ethanol, after which the recovered product is chromatographed on silica gel and the collected fraction subjected to treatment with ammonia to convert the lower alkyl ester, preferably the methyl or ethyl ester, to the C-terminal amide. In this manner the compounds of formula III in which X is hydrogen are obtained. The side chain protecting groups are then cleaved by procedures described above, for example by treatment with sodium in liquid ammonia or by hydrogen fluoride to give the corresponding compound of formula I wherein X is hydrogen.

The peptide compounds of formula I in which X is other than hydrogen are preferably obtained by acylation of the corresponding compound of formula II or III in which X is hydrogen with the appropriate acylating agent, followed by cleaving the resin support together with the anchoring radical, and all the remaining side chain protecting groups of the acylated product by the procedures described above. Preferably, a lower alkanoic acid chloride or bromide or lower alkanoic anhydride in the presence of an organic proton acceptor, e.g. pyridine or imidazole, is used as the acylating agent to prepare the compounds of formula II or III in which X is lower alkanoyl. Likewise, in the presence of an organic proton acceptor, compounds of formula II or III in which X is $HOOC{-}(CH_2)_n{-}CO$ are prepared using the acid chloride or acid bromide corresponding to the desired acyl radical X, e.g. succinyl chloride can be used for preparing compounds of formula II or III in which X is $HOOCCH_2CH_2CO$. The compound of formula II or III in which X is the acyl portion or N-acylated acyl portion of a D- or L-amino acid are prepared conveniently by the "activated ester" coupling procedure. Accordingly, the amino acid corresponding to the desired acyl portion to be incorporated into the compound of formula II or III is converted into an activated ester. Descriptions of such carboxyl-activating groups are found in general textbooks of peptide chemistry; for

example K. D. Kopple, "Peptides and Amino Acids", W. A. Benjamin, Inc., New York, 1966, pp. 45—51, and E. Schröder and K. Lübke, "The Peptides"; Vol. I, Academic Press, New York, 1965, pp. 77—128. Examples of the activated form of the terminal carboxyl are acid chloride, anhydride, azide, activated ester, or *O*-acyl urea of a dialkylcarbodiimide. The following activated esters have proved to be particularly suitable in the process of this invention: 2,4,5-trichlorophenyl (represented by Tcp), pentachlorophenyl (represented by Pep), *p*-nitrophenyl (represented by Np); the 1-benzotriazolyl group (represented by Bt) or the succinimido group is also useful for such activation.

The peptide compounds of formula I in which X is hydrogen also are useful for preparing the compounds of formula I in which X is other than hydrogen by subjecting the former compound to the appropriate acylating agent.

Although a solid phase synthesis of the compound of formula I is disclosed herein in detail, the preparation of the peptide also can be realized by classical solution methods well-known and described in detail in all textbooks, dealing with peptide chemistry.

Preparation of the resins

By way of example the preparation of some resins are described hereunder.

A. D - p - chlorophenylalanyl - D - p - chlorophenylalanyl - D - tryptophyl-L-seryl (O-benzyl) - L - tyrosyl - D - arginyl ($N^G$ - tosyl) - L - leucyl - L - arginyl ($N^G$ - tosyl) - L - prolyl - D - alanyl - benzhydrylamine resin;

Benzhydrylamine resin (1.05 g, 0.5 mmole) was placed in the reaction vessel of a Beckman Model 990 automatic peptide synthesizer programmed to carry out the following work-wash cycle: (a) methylene chloride; (b) 33% trifluoroacetic acid in methylene chloride (2 times for 1 and 25 min each); (c) methylenechloride; (d) ethanol; (e) methylene chloride; (f) 10% triethylamine in chloroform (2 times for 3 min. each); (g) methylene chloride.

The washed resin was stirred with t-butyloxycarbonyl D-alanine (320 mg, 1.5 mmoles) in methylene chloride and diisopropylcarbodiimide (1.5 mmoles) was added. The mixture was stirred at room temperature for 1 h and the amino acid resin was then washed with methylene chloride. The protected, attached amino acid was then cycled through steps (b) through (g) in the above wash program. The following amino acids (1.5 mmoles) were then coupled successively by the same cycle of events: t-Boc-proline, t-Boc-L-arginine ($N^G$-Tos), t-Boc-L-leucine; t-Boc-D-arginine ($N^G$-Tos), t-Boc-L-tyrosine, t-Boc-L-serine (O-Bzl), t-Boc-D-tryptophan, t-Boc-D-p-chlorophenylanine and t-Boc-D-p-chloro-phenylalanine.

Upon completion of the last coupling reaction the N-terminal t-Boc group was removed whereupon the resulting resin was washed with methanol and dried.

B. N - acetyl - D - p - chlorophenylalanyl - D - p - chlorophenylalanyl - D - tryptophyl - L - seryl (O - benzyl) - L - tyrosyl - D - arginyl ($N^G$ - tosyl) - L - leucyl - L - arginyl ($N^G$ - tosyl) - L - prolyl - D - alanyl - benzhydrylamine resin;

The resin obtained in A. was treated for 30 min. with 60 ml of a solution of imidazole (5 g) and acetic anhydride (3.54 ml) in methylene chloride (100 ml). The acetylated peptide-resin was then washed with methylene chloride and methanol. and dried.

C. D - p - chlorophenylalanyl - D - p - chlorophenylalanyl - D - tryptophyl - L - seryl (O - benzyl) - L - tyrosyl - D - lysyl ($N^\varepsilon$ - 2 - chlorobenzyloxycarbonyl) - L - leucyl - L - arginyl ($N^G$ - tosyl) - L - prolyl - D - alanyl - benzhydrylamine resin.

The resin was manufactured on the same scale and under the same conditions as described in A. with the exception that t-Boc-D-lysine ($N^\varepsilon$ - 2 - chlorobenzyloxycarbonyl) was incorporated in place of t-Boc-D-arginine ($N^G$-Tosyl).

D. The corresponding N-terminal acetyl derivative of the resin obtained in C. was prepared in the same manner as described in B.

E. D - 3 - (2 - Naphtyl) - alanyl - D - p - chlorophenylalanyl - D - tryptophyl - L - seryl - (O - benzyl) - L - tyrosyl - D - Arginyl ($N^G$ - tosyl) - L - leucyl - L - arginyl ($N^G$ - tyrosyl) - L - prolyl - D - alanyl - benzhydrylamine resin.

The resin was manufactured on the same scale and under the same conditions as described in A. with the exception that as the N-terminal amino acid was coupled t - Boc - D - 3 - (2 - naphtyl) - alanine in place of t - Boc - D - p - chloro - phenyl - alanine.

F. N - Acetyl - D - 3 - (2 - naphtyl) - alanyl - D - p - chlorophenylalanyl - D - tryptophyl - L - seryl - (O - benzyl) - L - tyrosyl - D - arginyl ($N^G$ - tosyl) - L - leucyl - L - arginyl ($N^G$ - tyrosyl) - L - prolyl - D - alanyl - benzhydrylamine resin.

The resin obtained in E. was acetylated as described in B. The acetylated peptide resin was then washed with methylenechloride and methanol and dried.

G. L - 3 - (2 - Naphtyl) - alanyl - D - p - chlorophenylalanyl - D - tryptophyl - L - seryl - (O - benzyl) - L - tyrosyl - D - lysyl ($N^{\varepsilon}$ - 2 - chlorobenzyloxycarbonyl) - L - leucyl - L - arginyl ($N^G$ - tosyl) - L - prolyl - D - alanyl - benzhydrylamine resin.

The resin was manufactured on the same scale and under the same conditions as described in E. with the exception that t - Boc - D - lysine ($N^{\varepsilon}$ - 2 - chlorobenzyloxycarbonyl) was incorporated in place of t - Boc - D - Arginine ($N^G$ - tosyl) and L - 3 - (2 - naphtyl) - alanine instead of D - 3 - (2 - naphtyl) - alanine.

H. The corresponding N-terminal acetyl derivative of the resin obtained in G. was prepared in the same manner as described in B.

K. The corresponding N-terminal-succinyl derivative of the resin described in A. was obtained by reacting 0.75 g of the resin A. with 30 ml of a solution of imidazol and succinylchloride in methylenechloride for 30 minutes. The acylated peptide-resin was then washed with methylene-chloride and methanol and dried in vacuo.

The following examples illustrate further this invention. In the examples, the ratio noted in connection with a mixture of solvents refers to the relative proportion of the solvents with respect to volume.

Example 1

N-Ac-[D-p-Cl-$Phe^{1,2}$, D-$Trp^3$, D-$Arg^6$, D-$Ala^{10}$]-LH-RH

Removal of the protecting groups (with the exception of the N-acetyl group) and cleavage of the decapeptide from the resin described previously (see A. and B.) was carried out by treatment of 1.96 g of the resin with hydrogen fluoride (40 ml), anisole (10 ml), and 1,4-dithiothreitol (100 mg) at 0°C for 1 h. The hydrogen fluoride was removed under a nitrogen stream and the peptide precipitated by addition of ether.

The crude peptide was extracted with 50% acetic acid. The extract was subjected to gel filtration on a column (2.5×95 cm) of a fine grade chemically modified cross-linked dextran, sold under the trade mark "Sephadex G-25", using 50% acetic acid as the solvent. Fractions shown to contain a major peak by UV absorption at 280 nm were pooled and evaporated to dryness. The residual oil was applied to a column (2.5×45 cm) of octadecylsilane bonded silica gel and eluted with a linear gradient of 10% to 40% n-propanol in 20% acetic acid 0.1 M ammonium acetate. The major peak was collected, evaporated to an oil and lyophilized in dilute acetic acid to give the title compound (232 mg) as a white powder. The product was homogeneous when examined by thin layer chromatography on silica gel in 4 different solvent systems when loads of 20—30 mcg were applied and spots visualized by $Cl_2$-starch reagent and Ehrlich reagent. The following $R_f$ values were obtained: (A) 1-butanol: acetic acid: water (4:1:1), 0.43; (B) ethyl acetate:pyridine:acetic acid:water (20:5:1:3), 0.12; (C) 2-propanol:1 M acetic acid (2:1), 0.43; (D) 1-butanol:acetic acid:water:ethyl acetate (1:1:1:1), 0.64. Amino acid analysis gave: Ser, 0.80; Pro, 0.97; Ala, 1.00; Leu, 1.03; Tyr, 1.00; p-Cl-Phe, 2.00; Trp, 0.66; Arg, 1.81.

Example 2

N-Ac-[D-3-(2-naphtyl)-$Ala^1$, D-p-Cl-$Phe^2$, D-$Trp^3$, D-$Arg^6$, D-$Ala^{10}$]-LH-RH

The title compound was prepared from the resin described under E. or F. in the way described in Example 1.

The crude peptide was purified using the same extraction and chromatographic methods as described in Example 1.

Example 3

N-Ac-[D-p-Cl-$Phe^{1,2}$, D-$Trp^3$, D-$Lys^6$, D-$Ala^{10}$]-LH-RH

Removal of the protecting groups (with the exception of the N-acetyl group and cleavage of the decapeptide from the resin (see D.) was accomplished under the same conditions as described in Example 1.

The crude peptide was purified using the same extraction and chromatographic methods as described in Example 1.

Example 4

N-Ac-[D-3-(2-naphtyl)-$Ala^1$, D-p-Cl-$Phe^2$, D-$Trp^3$, D-$Lys^6$, D-$Ala^{10}$]-LH-RH

Removal of the protecting groups (with the exception of the N-acetyl group and cleavage of the decapeptide from the resin (see H.) was accomplished under the same conditions as described in Example 1.

The crude peptide was purified using the same extraction and chromatographic methods as described in Example 1.

Example 5

In the same manner as described in Example 1 the following peptides were prepared:

[D-p-Cl-$Phe^{1,2}$, D-$Trp^3$, D-$Arg^6$, D-$Ala^{10}$]-LH-RH,
[D-p-Cl-$Phe^{1,2}$, D-$Trp^3$, D-$Lys^6$, D-$Ala^{10}$]-LH-RH,

N-Suc-[D-p-Cl-$Phe^{1,2}$, D-$Trp^3$, D-$Arg^6$, D-$Ala^{10}$]-LH-RH,
N-Ac-[D-p-Cl-$Phe^{1,2}$, D-$Trp^3$, D-$His^6$, D-$Ala^{10}$]-LH-RH,
N-Ac-[D-p-Cl-$Phe^{1,2}$, D-$Trp^3$, D-$Arg^6$]-LH-RH,
N-Ac-Gly-[D-p-Cl-$Phe^{1,2}$, D-$Trp^3$, D-$Arg^6$, D-$Ala^{10}$]-LH-RH,
N-Ac-[D-$Phe^1$, D-p-F-$Phe^2$, D-$Trp^3$, D-$Arg^6$, D-$Ala^{10}$]-LH-RH,
N-Ac-[D-p-Cl-$Phe^{1,2}$, D-$Trp^3$, D-$Orn^6$-D-$Ala^{10}$]-LH-RH,
N-Ac-[D-p-Cl-$Phe^{1,2}$, D-β-naphtyl-$Ala^3$, D-$Lys^6$-D-$Ala^{10}$]-LH-RH,
N-Ac-[D-$Trp^1$, D-p-Cl-$Phe^2$, D-$Trp^3$, D-$Arg^6$, D-$Ala^{10}$]-LH-RH,
N-Ac-[D-p-Cl-$Phe^{1,2}$, $Phe^3$, D-homo-$Arg^6$, D-$Ala^{10}$]-LH-RH,
[D-3-(2-naphtyl)-$Ala^1$, D-p-Cl-$Phe^2$, D-$Trp^3$, D-$Arg^6$, D-$Ala^{10}$]-LH-RH,
[D-3-(2-naphtyl)-$Ala^1$, D-p-Cl-$Phe^2$, D-$Trp^3$, D-$Lys^6$, D-$Ala^{10}$]-LH-RH,
[L-3-(2-naphtyl)-$Ala^1$, D-p-Cl-$Phe^2$, D-$Trp^3$, D-$Arg^6$, D-$Ala^{10}$]-LH-RH,
N-Ac-[L-3-(2-naphtyl)-$Ala^1$, D-p-F-$Phe^2$, D-$Trp^3$, D-$Arg^6$, D-$Ala^{10}$]-LH-RH,
N-Ac-[D-p-Cl-$Phe^2$, D-3-(2-naphtyl)-$Ala^{1,3}$, D-$Lys^6$, D-$Ala^{10}$]-LH-RH.

## Claims

1. A peptide of formula I

$$X\text{-}R^1\text{-}R^2\text{-}R^3\text{-Ser-Tyr-}R^4\text{-Leu-Arg-Pro-}R^5\text{-}NH_2 \quad (I)$$

in which X is hydrogen, lower alkanoyl, or HOOC—$(CH_2)_n$—CO wherein n is an integer from 2 to 6, $R^1$ is Gly, L-Ala, L-3-(1-naphtyl)-Ala, L-3-(2-naphtyl)-Ala, D-Ala, D-3-(1-naphtyl)-Ala, D-3-(2-naphtyl)-Ala, D-Trp, D-Phe or D-Phe having one or more substituents at the phenyl moiety selected from the group consisting of halogen, nitro, amino, alkyl (1—4 C), cyano, trifluoromethyl, hydroxy and alkoxy (1—4 C); $R^2$ is D-Phe having one or two substituents at the phenyl moiety, one substituent being always in para position which substituent(s) is (are) selected from the group consisting of halogen, nitro, amino, alkyl (1—4 C), cyano, trifluoromethyl, hydroxy and alkoxy (1—4 C); $R^3$ is D-Trp, L-Trp, L-Phe or L- or D-3-(2-naphtyl)-Ala; $R^4$ is D-Lys, D-Arg, D-Orn, D-homo-Arg or D-His and $R^5$ is Gly or D-Ala; or a therapeutically acceptable salt thereof.

2. The compound of Claim 1 in which X is hydrogen, lower alkanoyl, HOOC—$(CH_2)_n$—CO wherein n is an integer from 2 to 6, $R^1$ is D-3-(2-naphtyl)-Ala, L-3-(2-naphtyl)-Ala, D-Trp, D-Phe or D-p-Y-Phe wherein Y is selected from the group consisting of halogen, nitro, amino, methyl, cyano, trifluoromethyl, hydroxy and methoxy; $R^2$ is D-p-Y-Phe in which X is as defined herein; $R^3$ is D-Trp; $R^4$ is D-Lys or D-Arg and $R^5$ is D-Ala; or a therapeutically acceptable salt thereof.

3. The compound of Claim 1 in which X is hydrogen, lower alkanoyl or HOOC—$(CH_2)_n$—CO wherein n is an integer from 2 to 6; $R^1$ is D-3-(2-naphtyl)-Ala, D-Trp, D-Phe or D-p-halo-Phe; $R^2$ is D-p-halo-Phe; $R^3$ is D-Trp; $R^4$ is D-Lys or D-Arg and $R^5$ is D-Ala; or a therapeutically acceptable salt thereof.

4. The compound of Claim 1 in which X is acetyl or $HOOCCH_2$—$CH_2CO$, $R^1$ is D-3-(2-naphtyl)-Ala, D-Trp, D-Phe or D-p-Cl-Phe; $R^2$ is D-p-Cl-Phe; D-p-Br-Phe or D-p-F-Phe; $R^3$ is D-Trp; $R^4$ is D-Lys or D-Arg and $R^5$ is D-Ala; or a therapeutically acceptable salt thereof.

5. D - p - Chloro - phenylalanyl - D - p - chlorophenylalanyl - D - tryptophanyl - L - seryl - L - tyrosyl - D - arginyl - L - leucyl - L - arginyl - L - propyl - D - alaninamide, as claimed in Claim 1.

6. N - Acetyl - D - p - chlorophenylalanyl - D - p - chlorophenylalanyl - D - tryptophanyl - L - seryl - L - tyrosyl - D - arginyl - L - leucyl - L - arginyl - L - prolyl - D - alaninamide, as claimed in Claim 1.

7. D - p - Chloro - phenylalanyl - D - p - chlorophenylalanyl - D - tryptophanyl - L - seryl - L - tyrosyl - D - lysyl - L - leucyl - L - arginyl - L - prolyl - D - alaninamide, as claimed in Claim 1.

8. N - Acetyl - D - p - chlorophenylalanyl - D - p - chlorophenylalanyl - D - tryptophanyl - L - seryl - L - tyrosyl - D - lysyl - L - leucyl - L - arginyl - L - prolyl - D - alaninamide, as claimed in Claim 1.

9. N - Succinyl - D - p - chloro - phenylalanyl - D - p - chlorophenylalanyl - D - tryptophanyl - L - seryl - L - tyrosyl - D - arginyl - L - leucyl - L - arginyl - L - prolyl - D - alaninamide, as claimed in Claim 1.

10. N - Acetyl - D - p - chlorophenylalanyl - D - p - chlorophenylalanyl - D - tryptophyl - L - seryl - L - tyrosyl - D - histidyl - L - leucyl - L - arginyl - L - prolyl - D - alaninamide, as claimed in Claim 1.

11. D - 3 - (2 - Naphtyl) - alanyl - D - p - chlorophenylalanyl - D - tryptophanyl - L - seryl - L - tyrosyl - D - arginyl - L - leucyl - L - arginyl - L - prolyl - D - alaninamide, as claimed in Claim 1.

12. N - Acetyl - D - 3 - (2 - naphtyl) - alanyl - D - p - chlorophenylalanyl - D - tryptophanyl - L - seryl - L - tyrosyl - D - arginyl - L - leucyl - L - arginyl - L - prolyl - D - alaninamide, as claimed in Claim 1.

13. D - 3 - (2 - Naphtyl) - alanyl - D - p - chlorophenylalanyl - D - tryptophanyl - L - seryl - L - tyrosyl - D - lysyl - L - leucyl - L - arginyl - L - prolyl - D - alaninamide, as claimed in Claim 1.

14. N - Acetyl - D - 3 - (2 - naphtyl) - alanyl - D - p - chlorophenylalanyl - D - tryptophanyl - L - seryl - L - tyrosyl - D - lysyl - L - leucyl - L - arginyl - L - prolyl - D - alaninamide, as claimed in Claim 1.

15. A gonadotropin antagonizing pharmaceutical composition comprising a gonadotropin

antagonizing amount of the compound of Claim 1 or a therapeutically acceptable salt thereof, and a pharmaceutically acceptable carrier.

16. A gonadotropin antagonizing oral pharmaceutical composition comprising a gonadotropin antagonizing amount of the compound of Claim 1 or a therapeutically acceptable salt thereof, and a pharmaceutically acceptable carrier.

17. A compound of the formula II

$$X\text{-}R^1\text{-}R^2\text{-}R^3\text{-}Ser(R^6)\text{-}Tyr(R^7\text{-}R^{4(1)}\text{-}Leu\text{-}Arg(N^G\text{-}R^8)\text{-}Pro\text{-}R^5\text{-}A$$

wherein

X, $R^1$, $R^2$, $R^3$ and $R^5$ have the meanings defined in claim 1,

$R^{4(1)}$ has the same meanings as $R^4$ defined in claim 1 but in addition may also represent $N^\varepsilon$-protected D-Lys, $N^5$ protected D-Orn, $N^9$ protected D-Arg, $N^G$ protected D-homo-Arg or N' protected His,

$R^6$, $R^7$ and $R^8$ are each hydrogen or a protective group and

A represents

—NH—CH—Ph—Phe— resin support

or

$—OCH_2$—Ph— resin support .

## Patentansprüche

1. Peptid der Formel I:

$$X\text{-}R^1\text{-}R^2\text{-}R^3\text{-}Ser\text{-}Tyr\text{-}R^4\text{-}Leu\text{-}Arg\text{-}Pro\text{-}R^5\text{-}NH_2 \quad [I]$$

in welcher

X Wasserstoff, Nieder-Alkanoyl oder $HOOC—(CH_2)_n—CO$, worin n eine ganze Zahl von 2 bis 6 ist;

$R^1$ Gly, L-Ala, L-3-(1-Naphthyl)-Ala, L-3-(2-Naphtyl)-Ala, D-Ala, D-3-(1-Naptyl)-Ala, D-3-(2-Naphtyl)-Ala, D-Trp, D-Phe oder D-Phe mit einem oder mehreren Substituenten am Phenylteil, ausgewählt aus der Gruppe bestehend aus Halogen, Nitro, Amino, Alkyl (1—4 C), Cyano, Trifluormethyl, Hydroxy und Alkoxy (1—4 C);

$R^2$ D-Phe mit einem oder mehreren Substituenten am Phenylteil, wobei ein Substituent immer in para-Stellung steht und der bzw. die Substituent(en) ausgewählt ist bzw. sind aus der Gruppe bestehend aus Halogen, Nitro, Amino, Alkyl (1—4 C), Cyano, Trifluormethyl, Hydroxy und Alkoxy (1—4 C);

$R^3$ D-Trp, L-Trp, L-Phe oder L- oder D-3-(2-Naphtyl)-Ala;

$R^4$ D-Lys, D-Arg, D-Orn, D-Homo-Arg oder D-His und

$R^5$ Gly oder D-Ala bedeutet; oder ein therapeutisch annehmbares Salz davon.

2. Verbindung nach Patentanspruch 1, in welcher X Wasserstoff, Nieder-Alkanoyl, $HOOC—(CH_2)_n—CO$, worin n eine ganze Zahl von 2 bis 6 ist; $R^1$ D - 3 - (2 - Naphtyl) - Ala, L - 3 - (2 - Naphtyl) - Ala, D-Trp, D-Phe oder D-p-Y-Phe, worin Y ausgewählt ist aus der Gruppe bestehend aus Halogen, Nitro, Amino, Methyl, Cyano, Trifluormethyl, Hydroxy und Methoxy; $R^2$ D-p-Y-Phe, worin Y dieselbe Bedeutung wie oben hat; $R^3$ D-Trp; $R^4$ D-Lys oder D-Arg und $R^5$ D-Ala bedeutet, oder ein therapeutisch annehmbares Salz davon.

3. Verbindung nach Patentanspruch 1, in welcher X Wasserstoff, Niederalkanoyl oder $HOOC—(CH_2)_n—CO$, worin n eine ganze Zahl von 2 bis 6 ist; $R^1$ D - 3 - (2 - Naphtyl) - Ala, D-Trp, D-Phe oder D-p-Halogen-Phe; $R^2$ D-p-Halogen-Phe; $R^3$ D-Trp; $R^4$ D-Lys oder D-Arg und $R^5$ D-Ala bedeutet, oder ein therapeutisch annehmbares Salz davon.

4. Verbindung nach Patentanspruch 1, in welcher X Acetyl oder $HOOCCH_2—CH_2CO$, $R^1$ D - 3 - (2 - Naphtyl) - Ala, D-Trp, D-Phe oder D-p-Cl-Phe; $R^2$ D-p-Cl-Phe; D-p-Br-Phe oder D-p-F-Phe; $R^3$ D-Trp; $R^4$ D-Lys oder D-Arg und $R^5$ D-Ala bedeutet oder ein therapeutisch annehmbares Salz davon.

5. D - p - Chlor - phenylalanyl - D - p - chlorphenylalanyl - D - tryptophanyl - L - seryl - L - tyrosyl - D - arginyl - L - leucyl - L - arginyl - L - propyl - D - alanin - amid als Verbindung nach Patentanspruch 1.

6. N - Acetyl - D - p - chlorphenylalanyl - D - p - chlorphenylalanyl - D - tryptophanyl - L - seryl - L - tyrosyl - D - arginyl - L - leucyl - L - arginyl - L - prolyl - D - alaninamid als Verbindung nach Patentanspruch 1.

7. D - p - Chlor - phenylalanyl - D - p - chlorphenylalanyl - D - tryptophanyl - L - seryl - L - tyrosyl - D - lysyl - L - leucyl - L - arginyl - L - prolyl - D - alaninamid als Verbindung nach Patentanspruch 1.

8. N - Acetyl - D - p - chlorphenylalanyl - D - p - chlorphenylalanyl - D - tryptophanyl - L - seryl - L - tyrosyl - D - lysyl - L - leucyl - L - arginyl - L - prolyl - D - alaninamid als Verbindung nach Patentanspruch 1.

9. N - Succinyl - D - p - chlor - phenylalanyl - D - p - chlorphenylalanyl - D - tryptophanyl - L - seryl - L - tyrosyl - D - arginyl - L - leucyl - L - arginyl - L - prolyl - D - alaninamid als Verbindung nach Patentanspruch 1.

10. N - Acetyl - D - p - chlorphenylalanyl - D - p - chlorphenylalanyl - D - tryptophyl - L - seryl -

L - tyrosyl - D - histidyl - L - leucyl - L - arginyl - L - prolyl - D - alaninamid als Verbindung nach Patentanspruch 1.

11. D - 3 - (2 - Naphtyl) - alanyl - D - p - chlorphenylalanyl - D - tryptophanyl - L - seryl - L - tyrosyl - D - arginyl - L - leucyl - L - arginyl - L - prolyl - D - alaninamid als Verbindung nach Patentanspruch 1.

12. N - Acetyl - D - 3 - (2 - naphtyl) - alanyl - D - p - chlorphenylalanyl - D - tryptophanyl - L - seryl - L - tyrosyl - D - arginyl - L - leucyl - L - arginyl - L - prolyl - D - alaninamid als Verbindung nach Patentanspruch 1.

13. D - 3 - (2 - Naphtyl) - alanyl - D - p - chlorphenylalanyl - D - tryptophanyl - L - seryl - L - tyrosyl - D - lysyl - L - leucyl - L - arginyl - L - prolyl - D - alaninamid als Verbindung nach Patentanspruch 1.

14. N - Acetyl - D - 3 - (2 - naphtyl) - alanyl - D - p - chlorophenylalanyl - D - tryptophanyl - L - seryl - L - tyrosyl - D - lysyl - L - leucyl - L - arginyl - L - prolyl - D - alaninamid als Verbindung nach Patentanspruch 1.

15. Gonadotropin antagonisierendes, pharmazeutisches Präparat, welches eine Gonadotropin antagonisierende Menge der Verbindung nach Patentanspruch 1 oder eines therapeutisch annehmbaren Salzes davon und einen pharmazeutisch annehmbaren Träger enthält.

16. Gonadotropin antagonisierendes orales pharmazeutisches Präparat, welches eine Gonadotropin antagonisierende Menge der Verbindung nach Patentanspruch 1 oder eines therapeutisch annehmbaren Salzes davon und einen pharmazeutisch annehmbaren träger enthält.

17. Verbindung der Formel II:

$$X\text{-}R^1\text{-}R^2\text{-}R^3\text{-Ser}(R^6)\text{-Tyr}(R^7\text{-}R^{4(1)}\text{-Leu-Arg}(N^G\text{-}R^8)\text{-Pro-}R^5\text{-A}$$

in welcher X, $R^1$, $R^2$, $R^3$ und $R^5$ doeseöbe Bedeutung wie in Patentanspruch 1 aufweisen; $R^{4(1)}$ dasselbe wie $R^4$ in Patentanspruch 1 bedeutet und zusätzlich $N^\varepsilon$-geschütztes D-Lys, $N^\delta$-geschütztes D-Orn, $N^g$-geschütztes D-Arg, $N^G$-geschütztes D-Homo-Arg oder $N^I$-geschütztes His sein kann; $R^6$, $R^7$ und $R^8$ jedes Wasserstoff oder eine Schutzgruppe darstellt, und A

—NH—CH—Ph—Phe— | Harzunterlage |

oder

—$OCH_2$—Phe— | Harzunterlage |

bedeutet.

## Revendications

1. Un peptide de formule I

$$X\text{-}R^1\text{-}R^2\text{-}R^3\text{-Ser-Tyr-}R^4\text{-Leu-Arg-Pro-}R^5\text{-}NH_2 \qquad (I)$$

dans laquelle

X est de l'hydrogène, un groupe alcanoyle inférieur, ou $HOOC—(CH_2)_n—CO$ où n est un nombre entier de 2 à 6,

$R^1$ est Gly, L-Ala, L - 3 - (1 - naphtyl) - Ala, L - 3 - (2 - naphtyl) - Ala, D-Ala, D - 3 - (1 - naphtyl) - Ala, D - 3 - (2 - naphtyl) - Ala, D-Trp, D-Phe ou D-Phe ayant un ou plusieurs substituants au niveau du motif phényle choisis dans la classe formée par les groupes halogéno, nitro, amino, alkyle ($C_1—C_4$), cyano, trifluorométhyle, hydroxy et alcoxy ($C_1—C_4$);

$R^2$ est D-Phe ayant un ou deux substituants au niveau du motif phényle, un substituant étant toujours en position para, ce ou ces substituants étant choisis dans la classe formée par les groupes halogéno, nitro, amino, alkyle ($C_1—C_4$), cyano, trifluorométhyle, hydroxy et alcoxy ($C_1—C_4$);

$R^3$ est D-Trp, L-Trp, L-Phe ou L- ou D-3-(2-naphtyl)-Ala;

$R^4$ est D-Lys, D-Arg, D-Orn, D-homo-Arg ou D-His et

$R^5$ est Gly ou D-Ala; ou un sel thérapeutiquement acceptable de celui-ci.

2. Le composé de la revendication 1 dans lequel

X est l'hydrogène, un groupe alcanoyle inférieur, $HOOC—(CH_2)_n—CO$; où

n est un nombre entier de 2 à 6,

$R^1$ est D - 3 - (2 - naphtyl) - Ala, L - 3 - (2 - naphtyl) - Ala, D-Trp, D-Phe ou D-p-Y-Phe; où

Y est choisi dans la classe formée des groupes halogéno, nitro, amino, méthyle, cyano, trifluorométhyle, hydroxy et méthoxy;

$R^2$ est D-p-Y-Phe où

X est comme défini ci-dessus;

$R^3$ est D-Trp;

$R^4$ est D-Lys ou D-Arg et

$R^5$ est D-Ala; ou un sel thérapeutiquement acceptable de celui-ci.

3. Le composé de la revendication 1 dans lequel
X est l'hydrogène, un groupe alcanoyle inférieur ou HOOC—$(CH_2)_n$—CO où n est un nombre entier de 2 à 6;
$R^1$ est D-3-(2-naphtyl)-Ala, D-Trp, D-Phe ou D-p-halo-Phe;
$R^2$ est D-p-halo-Phe;
$R^3$ est D-Trp;
$R^4$ est D-Lys ou D-Arg et
$R^5$ est D-Ala; ou un sel thérapeutiquement acceptable de celui-ci.

4. Le composé de la revendication 1 dans lequel
X est un groupe acétyle ou $HOOCCH_2$—$CH_2CO$,
$R^1$ est D-3-(2-naphtyl)-Ala, D-Trp, D-Phe ou D-p-Cl-Phe;
$R^2$ est D-p-Cl-Phe; D-p-Br-Phe ou D-p-F-Phe;
$R^3$ est D-Trp;
$R^4$ est D-Lys ou D-Arg et
$R^5$ est d-Ala; ou un sel thérapeutiquement acceptable de celui-ci.

5. Le D - p - chloro - phénylalanyl - D - p - chlorophényl - alanyl - D - tryptophanyl - L - séryl - L - tyrosyl - D - arginyl - L - leucyl - L - arginyl - L - propyl - D - alanine - amide, selon la revendication 1.

6. Le N - acétyl - D - p - chlorophénylalanyl - D - p - chloro - phénylalanyl - D - tryptophanyl - L - séryl - L - tyrosyl - D - arginyl - L - leucyl - L - arginyl - L - prolyl - D - alaninamide, selon la revendication 1.

7. Le D - p - chloro - phényl - alanyl - D - p - chlorophényl - alanyl - D - tryptophanyl - L - séryl - L - tyrosyl - D - lysyl - L - leucyl - L - arginyl - L - prolyl - D - alaninamide, selon la revendication 1.

8. Le N - acétyl - D - p - chlorophénylalanyl - D - p - chlorophénylalanyl - D - tryptophanyl - L - séryl - L - tyrosyl - D - lysyl - L - leucyl - L - arginyl - L - prolyl - D - alaninamide, selon la revendication 1.

9. Le N - succinyl - D - p - chloro - phénylalanyl - D - p - chlorophénylalanyl - D - tryptophanyl - L - séryl - L - tyrosyl - D - arginyl - L - leucyl - L - arginyl - L - prolyl - D - alaninamide, selon la revendication 1.

10. Le N - acétyl - D - p - chlorophénylalanyl - D - p - chlorophénylalanyl - D - tryptophanyl - L - séryl - L - tyrosyl - D - histidyl - L - leucyl - L - arginyl - L - prolyl - D - alaninamide, selon la revendication 1.

11. Le D - 3 - (2 - naphtyl) - alanyl - D - p - chlorophényl - alanyl - D - tryptophanyl - L - séryl - L - tyrosyl - D - arginyl - L - leucyl - L - arginyl - L - prolyl - D - alaninamide, selon la revendication 1.

12. Le N - acétyl - D - 3 - (2 - naphtyl) - alanyl - D - p - chlorophénylalanyl - D - tryptophanyl - L - séryl - L - tyrosyl - D - arginyl - L - leucyl - L - arginyl - L - prolyl - D - alaninamide, selon la revendication 1.

13. Le D - 3 - (2 - napthyl) - alanyl - D - p - chlorophényl - alanyl - D - tryptophanyl - L - séryl - L - tyrosyl - D - lysyl - L - leucyl - L - arginyl - L - prolyl - D - alaninamide, selon la revendication 1.

14. Le N - acétyl - D - 3 - (2 - naphtyl) - alanyl - D - p - chlorophénylalanyl - D - tryptophanyl - L - séryl - L - tyrosyl - D - lysyl - L - leucyl - L - arginyl - L - prolyl - D - alaninamide, selon la revendication 1.

15. Une composition pharmaceutique antagoniste de gonadotrophines comprenant une quantité, à action antigonadotrophines, du composé de la revendication 1 ou d'un sel thérapeutiquement acceptable de ce composé, et un support pharmaceutiquement acceptable.

16. Une composition pharmaceutique orale antagoniste de gonadotrophines comprenant une quantité, à action antigonadotrophines, du composé de la revendication 1 ou d'un sel thérapeutiquement acceptable de celui-ci, et un support pharmaceutiquement acceptable.

17. Un composé de formule II

$$X\text{-}R^1\text{-}R^2\text{-}R^3\text{-Ser}(R^6)\text{-Tyr}(R^7)\text{-}R^{4(1)}\text{-Leu-Arg}(N^G\text{-}R^8)\text{-Pro-}R^5\text{-A}$$

dans laquelle
X, $R^1$, $R^2$, $R^3$ et $R^5$ ont les significations définies dans la revendication 1,
$R^{4(1)}$ a les mêmes significations que $R^4$ défini dans la revendication 1, mais il peut en outre représenter également D-Lys protégé sur $N^\varepsilon$, D-Orn protégé sur $N^\delta$, D-Arg protégé sur $N^g$, D-homo-Arg protégé sur $N^G$ ou His protégé sur $N^I$,
$R^6$, $R^7$ et $R^8$ sont chacun de l'hydrogène ou un groupe protecteur et
A représente

—NH—CH—Ph—Phe— | support de résine |

ou

—$OCH_2$—Ph— | support de résine |.